# DEMANDE DE BREVET EUROPEEN

(11) **EP 1 498 109 A1**
(43) Date de publication de la demande: **19.01.2005**
(21) Numéro de dépôt: 04291688.2
(22) Date de dépôt: 02.07.2004
(51) Int. Cl.: A61K 7/13, C07D 417/12, C07D 307/68, C07D 405/12, C07D 307/71, C07D 307/73

(54) **Dérivés du furane et composition tinctoriale comprenant à titre de coupleur un dérivé du furane**

(30) Priorité: 15.07.2003 FR 0308604
(71) Demandeur: L'OREAL, 75008 Paris (FR)
(72) Inventeur: Mavro, Jacqueline, 94170 Le Perreux (FR); Vidal, Laurent, 75013 Paris (FR); Saunier, Jean-Baptiste, 75014 Paris (FR)
(74) Mandataire: Fevrier, Murielle

(57) **Abrégé**

L'invention a pour objet une composition tinctoriale utile pour la teinture des fibres kératiniques contenant au moins une base d'oxydation et au moins un coupleur du type furane, l'utilisation de cette composition pour la teinture des fibres kératiniques ainsi que le procédé de teinture mettant en oeuvre cette composition.

L'invention a aussi pour objet de nouveaux dérivés du furane utiles comme coupleurs pour la teinture par oxydation des fibres kératiniques.

## Description

L'invention a pour objet de nouvelles compositions tinctoriales comprenant dans un milieu approprié, au moins une base d'oxydation et au moins un coupleur du type furane.

Il est connu de teindre les fibres kératiniques et en particulier les cheveux humains avec des compositions tinctoriales contenant des précurseurs de colorant d'oxydation, en particulier des ortho ou para-phénylènediamines, des ortho ou para-aminophénols, des composés hétérocycliques tels que des dérivés de diaminopyrazole, des dérivés de pyrazolo[1,5-a]pyrimidine, des dérivés de pyrimidines, des dérivés de pyridine, des dérivés de 5,6-dihydroxyindole, des dérivés de 5,6-dihydroxyindoline appelés généralement bases d'oxydation. Les précurseurs de colorants d'oxydation, ou bases d'oxydation, sont des composés incolores ou faiblement colorés qui, associés à des produits oxydants, peuvent donner naissance par un processus de condensation oxydative à des composés colorés et colorants.

On sait également que l'on peut faire varier les nuances obtenues avec ces bases d'oxydation en les associant à des coupleurs ou modificateurs de coloration, ces derniers étant choisis notamment parmi les méta-phénylènediamines, les méta-aminophénols, les méta-hydroxyphénols et certains composés hétérocycliques tels que par exemple des dérivés de pyrazolo[1,5-b]-1,2,4,-triazoles, des dérivés de pyrazolo[3,2-c]-1,2,4,-triazoles, des dérivés de pyrazolo[1,5-a]pyrimidines, des dérivés de pyridine, des dérivés de pyrazol-5-one, des dérivés d'indoline et des dérivés d'indole.

La variété des molécules mises en jeu au niveau des bases d'oxydation et des coupleurs permet l'obtention d'une riche palette de couleurs.

La coloration dite "permanente" obtenue grâce à ces colorants d'oxydation, doit par ailleurs satisfaire un certain nombre d'exigences. Ainsi, elle doit être sans inconvénient sur le plan toxicologique, elle doit permettre d'obtenir des nuances dans l'intensité souhaitée, présenter une bonne tenue face aux agents extérieurs (lumière, intempéries, lavage, ondulation permanente, transpiration, frottements).

Les colorants doivent également permettre de couvrir les cheveux blancs, et être enfin les moins sélectifs possibles, c'est à dire permettre d'obtenir des écarts de coloration les plus faibles possibles tout au long d'une même fibre kératinique, qui peut être en effet différemment sensibilisée (i.e. abîmée) entre sa pointe et sa racine. Ils doivent également présenter une bonne stabilité chimique dans les formulations. Ils doivent présenter un bon profil toxicologique.

Le but de la présente invention est de fournir de nouveaux coupleurs permettant d'obtenir une gamme encore plus variée de nuances ainsi que des compositions tinctoriales qui présentent des teintures puissantes, uniformes entre la racine et la pointe des cheveux, ayant une bonne chromaticité, peu sélectives et particulièrement résistantes, tout en étant capables d'engendrer des colorations intenses dans des nuances variées, en particulier dans les nuances fondamentales.

Ce but est atteint avec la présente invention qui a pour objet une composition tinctoriale comprenant, dans un milieu approprié pour la teinture, au moins une base d'oxydation et au moins un coupleur de type furane de formule (I) suivante ainsi que ses sels d'addition avec un acide ou une base : 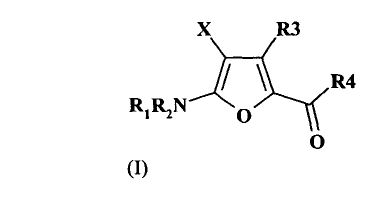 dans laquelle :
- R₁, R₂ représentent, identiques ou différents,
   - un atome d'hydrogène ;
   - un radical phényle éventuellement substitué par un ou plusieurs radicaux choisis parmi les halogènes, les radicaux hydroxy, alcoxy en C₁-C₄, carboxy, mono ou di alkyl(C₁-C₄)carboxamido (Alkyl (C₁-C₄)NHCO- ou Alkyl(C₁-C₄)₂NCO-), alkyl(C₁-C₄)sulfonyle (-SO₂alkyle), sulfonique (-SO₃H), alkylsulfoxyde (-SO-alkyle), alkylsulfonamido (alkyl(C₁-C₄)SO₂NH-), NR₁₃R₁₄, alkyle en C₁-C₄ éventuellement substitué parmi un ou plusieurs radicaux hydroxy, alcoxy en C₁-C₄, carboxy, mono ou di alkyl(C₁-C₄)carboxamido, alkyl(C₁-C₄)sulfonyle, sulfonique, alkylsulfoxyde, alkylsulfonamido ou NR₁₃R₁₄ ;
   - un radical alkyle en C₁-C₈ linéaire ou ramifié, éventuellement substitué par un ou plusieurs radicaux hydroxy, alcoxy en C₁-C₄, carboxy, mono ou di alkyl(C₁-C₄)carboxamido, alkyl(C₁-C₄)sulfonyle, sulfonique, alkylsulfoxyde, alkylsulfonamido ou NR₁₃R₁₄,
- R₁ et R₂ ensemble peuvent former avec l'atome d'azote avec lequel ils sont attachés un hétérocycle comportant 5 à 8 chaînons dont un ou plusieurs atomes de carbone du cycle carboné peuvent être remplacés par un atome d'oxygène, d'azote, de soufre ou par un groupement SO₂ ; les atomes de carbone dudit cycle peuvent être substitués par un radical R₅ ; le cycle ne comportant pas de liaison peroxyde, ni de radicaux diazo ou nitroso ;
   - R₅ représente :
      - un atome d'halogène ;
      - un radical alkyle en C₁-C₄ éventuellement substitué par un ou plusieurs radicaux hydroxy, carboxy, alcoxycarbonyle en C₁-C₄, alcoxy en C₁-C₄, ou NR₆R₇ ;
      - un radical carboxy ;
      - un radical mono ou di alkylcarboxamido ;
      - un radical alkyl(C1-C4)sulfonyle ;
      - un radical alkylsulfonamido
      - un radical hydroxy ;
      - un radical alcoxy en C₁-C₄ ;
      - un radical hydroxyalcoxy en C₂-C₄ ;
      - un radical aminosulfonyle (NH₂-SO₂-);
      - un radical thioéther en C₁-C₄ ;
      - un radical alkyl(C₁-C₄)sulfoxyde ;
      - un radical alkyl(C1-C4)sulfonyle ;
      - un radical NR₈R₉ ;
- R₃ représente
   - un atome d'hydrogène ;
   - un radical alkyl(C₁-C₄)sulfonyle;
   - un radical alkyle en C₁-C₈ linéaire ou ramifié, éventuellement substitué par un ou plusieurs radicaux hydroxy, OR₁₀, carboxy, alcoxycarbonyle en C₁-C₄, mono ou di alkyl(C₁-C₄)carboxamido, alkyl(C₁-C₄)sulfonyle, sulfonique, alkyl(C₁-C₄)sulfoxyde, alkylsulfonamido (alkyl(C₁-C₄)SO₂NH-) ou NR₁₁R₁₂ ;
   - un radical phényle ou un hétérocycle aromatique à 5 ou 6 chaînons tels que pyridyle, pyrimidinyle, pyrazolyle, thiazolyle, furyle, imidazolyle, thiènyle, ces radicaux étant éventuellement substitués par un ou plusieurs radicaux choisis parmi les halogène, les radicaux hydroxy, alcoxy en C₁-C₄, carboxy, alcoxycarbonyle en C₁-C₄, mono ou di alkyl(C₁-C₄)carboxamido, alkyl(C₁-C₄)sulfonyle, sulfonique, alkylsulfoxyde, thioéther en C₁-C₄, alkylsulfonamido, NR₁₃R₁₄, alkyle en C₁-C₄ éventuellement substitué par un ou plusieurs radicaux hydroxy, alcoxy en C₁-C₄, carboxy, mono ou di alkyl(C₁-C₄)carboxamido, alkyl(C₁-C₄)sulfonyle , sulfonique, alkylsulfoxyde, alkylsulfonamido ou NR₁₃R₁₄ ;
   - R₁₀ représente un radical alkyle en C₁-C₄ éventuellement substitué par un ou plusieurs radicaux hydroxy, alcoxy en C₁-C₂, amino, mono ou di alkyl (C₁-C₄)amino, (poly)hydroxyalkylamino en C₂-C₄,
   - R₆, R₇, R₈, R₉, R₁₁, R₁₂, R₁₃, R₁₄, représentent, identiques ou différents, un atome d'hydrogène, un radical sulfino, un radical alkyl(C₁-C₄)-CO , un radical mono ou di alkyl(C₁-C₄)carboxamido, un radical alkyle en C₁-C₄ éventuellement substitué par un ou plusieurs radicaux hydroxy, alcoxy en C₁-C₄, alkyl(C₁-C₄)sulfonyle, alkylsulfonamido, carboxy, mono ou di alkyl(C₁-C₄)carboxamido, alkylsulfoxyde, amino, mono- ou di-(C₁-C₄)alkylamino, (poly)hydroxyalkylamino en C₂-C₄ ;
   - R₈ et R₉ peuvent également former avec l'atome d'azote sur lequel ils sont attachés un cycle de 5 à 8 chaînons éventuellement substitué par un ou plusieurs radicaux choisis parmi les halogènes, les radicaux hydroxy, alcoxy en C₁-C₂ carboxy, alkylsulfonamido, NR₁₃R₁₄, alkyle en C₁-C₄ éventuellement substitué par un ou plusieurs radicaux hydroxy, alcoxy en C₁-C₄, carboxy, mono ou di alkyl(C₁-C₄)carboxamido, alkyl(C₁-C₄)sulfonyle, sulfonique, alkylsulfoxyde, alkylsulfonamido ou NR₁₃R₁₄;
- R₄ représente
   - un radical phényle éventuellement substitué par un ou plusieurs radicaux choisis parmi les halogène, les radicaux hydroxy, alcoxy en C₁-C₄, carboxy, alcoxycarbonyle en C₁-C₄, mono ou di alkyl(C₁-C₄)carboxamido, alkyl(C₁-C₄)sulfonyle, sulfonique, alkylsulfoxyde, thioéther en C₁-C₄, alkylsulfonamido, NR₁₃R₁₄, alkyle en C₁-C₄ éventuellement substitué par un ou plusieurs radicaux hydroxy, alcoxy en C₁-C₄, carboxy, mono ou di alkyl(C₁-C₄)carboxamido, alkyl(C₁-C₄)sulfonyle , sulfonique, alkylsulfoxyde, alkylsulfonamido ou NR₁₃R₁₄,
   - un radical hétéroaromatique choisi parmi un radical furyle, thiényle, oxazole, imidazole, thiazole, pyrrole, isoxazole, triazole, thiadiazole, pyridyle, pyrimidyle, pyrazinyle, pyridazinyle; éventuellement substitué un ou plusieurs radicaux choisis parmi les halogènes, les radicaux hydroxy, alcoxy en C₁-C₄, carboxy, alcoxycarbonyle en C₁-C₄, mono ou di alkyl(C₁-C₄)carboxamido, alkyl(C₁-C₄)sulfonyle, sulfonique, alkylsulfoxyde, thioéther en C₁-C₄, alkylsulfonamido, NR₁₃R₁₄, alkyle en C₁-C₄ éventuellement substitué par un ou plusieurs radicaux hydroxy, alcoxy en C₁-C₄, carboxy, mono ou di alkyl(C₁-C₄)carboxamido, alkyl(C₁-C₄)sulfonyle , sulfonique, alkylsulfoxyde, alkylsulfonamido ou NR₁₃R₁₄
   - un radical OR₁₀ ;
   - un radical NR₁₅R₁₆ ;
   - un radical OH ;
   - un radical alkyle en C₁-C₈ linéaire ou ramifié pouvant être substitué par un ou plusieurs radicaux hydroxy, OR₁₀, carboxy, alcoxycarbonyle, mono ou di alkyl(C₁-C₄)carboxamido, alkylsulfonyle, sulfino, alklsulfoxyde, alkylsulfonamido, NR₂₅R₂₆,
   - R₁₅, R₁₆, R₂₅ et R₂₆ représentent indépendamment l'un de l'autre un atome d'hydrogène, un radical phényle, un radical hétéroaromatique, un radical alkyle en C₁-C₆, les radicaux R₁₅, R₁₆, R₂₅ et R₂₆ étant éventuellement substitué par un ou plusieurs radicaux hydroxy, alcoxy en C₁-C₂, phényle, alkyl(C₁-C₄)sulfonamido, carboxy, mono ou di alkyl(C₁-C₄)carboxamido, alkyl(C₁-C₄)sulfonyle, alkyl(C₁-C₄)sulfoxyde, amino, mono- ou di- alkylamino, (poly)hydroxyalkylamino en C₂-C₄,
- X représente un atome d'hydrogène ; un atome d'halogène (tel que fluor, chlore, brome) ; un radical alcoxy en C₁-C₄ ; un radical phénoxy éventuellement substitué par un ou plusieurs radicaux choisis parmi les halogènes (tel que fluor, chlore, brome), les radicaux hydroxy, alcoxy en C₁-C₄, carboxy, alcoxycarbonyle en C₁-C₄, mono ou di alkyl(C₁-C⁴)carboxamido, alkyl(C₁-C₄)sulfonyle, sulfonique, alkylsulfoxyde, thioéther en C₁-C₄, alkylsulfonamido, N₁₃R₁₄, alkyle en C₁-C₄ éventuellement substitué par un hydroxy, alcoxy en C1-C2, carboxy, sulfonique, amino.

Les compositions de la présente invention conduisent à des colorations particulièrement puissantes et peu sélectives. Elles permettent de plus d'obtenir des colorations résistant bien aux diverses agressions que peuvent subir les cheveux, telles que lumière, intempéries, lavage, transpiration. Les compositions de teinture d'oxydation conformes à l'invention permettent de plus d'atteindre des nuances dans une très large palette de couleurs

La présente invention a également pour objet l'utilisation des composés de formule (I) pour la coloration des fibres kératiniques, en particulier les cheveux.

Un autre objet de l'invention est un procédé de teinture mettant en oeuvre la composition de la présente invention.

L'invention a aussi pour objet les composés nouveaux de formule (I).

Dans les radicaux définis ci-dessus porteurs de groupe alkyle et sauf indication différente, le radical alkyle peut être linéaire ou ramifié, et éventuellement substitué par un hydroxy, alcoxy ou carboxy. De plus, lorsque la molécule contient plusieurs radicaux de même dénomination, par exemple NR₁₃R₁₄, ces radicaux peuvent être identiques ou différents.

A titre d'exemple, on peut citer pour les radicaux R₁ et R₂ de la formule (I) un atome d'hydrogène, un radical méthyle, éthyle, (iso)propyle, 2-hydroxyéthyle, 1-hydroxyéthyle, 2-carboxyéthyle, 2-aminoéthyle, 2-(diméthylamino)éthyle, 2-(acylamino)éthyle, 2-méthoxyéthyle, 1,2-dihydroxyéthyle, 1-hydroxy-2-aminoéthyle, 2-hydroxy-1-aminoéthyle, acétyle, ou phényle.

Selon un mode de réalisation préférée, R₁ et R₂ représentent, identiques ou différents, un atome d'hydrogène ; un radical phényle ; un radical alkyle en C₁-C₄, linéaire ou ramifié éventuellement substitué par un ou plusieurs radicaux hydroxy, alcoxy en C₁-C₂, amino, mono ou di(C₁-C₄)alkylamino , carboxy, mono ou di alkyl(C₁-C₄)carboxamido, sulfonamido, NR₁₃R₁₄ où R₁₃ et R₁₄ représentent, identiques ou différents, un atome d'hydrogène, acyle, un radical alkyle en C₁-C₂ éventuellement substitué par un hydroxy ou alcoxy en C₁-C₂.

R₁ et R₂, identiques ou différents, représentent, de préférence, un atome d'hydrogène, les radicaux méthyle, 2-hydroxyéthyle, 2-carboxyéthyle ou 1,2-dihydroxyéthyle.

Selon un autre mode de réalisation, R₁ et R₂ forment avec l'atome d'azote avec lequel ils sont attachés un hétérocycle de 5 à 8 chaînons choisi parmi les pyrrolidine, pipéridine, homopipéridine, pipérazine, homopipérazine, lesdits cycles pouvant être substitués par un ou plusieurs radicaux R₅.

A titre d'exemple de ce mode de réalisation particulier, R₁ et R₂ forment ensemble avec l'atome d'azote auquel ils sont rattachés un hétérocycle choisi parmi la pyrrolidine, la 2,5-diméthylpyrrolidine, la proline, la 3-hydroxyproline, la 4-hydroxyproline, la 2,4-dicarboxypyrrolidine, la 3-hydroxy-2-hydroxyméthylpyrrolidine, la 2-carboxamidopyrrolidine, la 3-hydroxy-2-carboxamidopyrrolidine, la 2-(diéthylcarboxamido)pyrrolidine, la 2-hydroxyméthyl pyrrolidine, la 3,4-dihydroxy-2-hydroxyméthyl pyrrolidine, la 3-hydroxypyrrolidine, la 3,4-dihydroxy pyrrolidine, la 3-amino pyrrolidine, la 3-méthylamino pyrrolidine, la 4-amino-3-hydroxy pyrrolidine, la 3-hydroxy-4-(2-hydroxyéthyl)amino-pyrrolidine, la pipéridine, la 2,6-diméthylpipéridine, la 2-carboxypipéridine, la 2-carboxamidopipéridine, la 2-hydroxyméthylpipéridine, la 3-hydroxy-2-hydroxyméthylpipéridine, 3-hydroxypipéridine, la 4-hydroxypipéridine, la 3-hydroxyméthylpipéridine, l'homopipéridine, la 2-carboxyhomopipéridine, la 2-carboxamidohomopipéridine, l'homopiperazine, la N-méthyl homopipérazine, la N β-hydroxyéthylhomopipérazine et leurs sels d'addition.

De préférence, R₁ et R₂ forment ensemble avec l'atome d'azote auquel ils sont rattachés un hétérocycle choisi parmi la pyrrolidine, la 3-hydroxypyrrolidine, la 3-aminopyrrolidine, la proline, la 3-hydroxyproline, la pipéridine, la 3-hydroxypipéridine, la 3-hydroxypipéridine, l'homopipéridine, l'homopipérazine, la N-méthyl homopipérazine, la N β-hydroxyéthylhomopipérazine et leurs sels d'addition.

A titre d'exemple pour R3, on peut citer un atome d'hydrogène, un radical méthyle, éthyle, (iso)propyle, 2-hydroxyéthyle, 1-hydroxyéthyle, 2-carboxyéthyle, 2-aminoéthyle, 2-(diméthylamino)éthyle, 2-(acylamino)éthyle, 2-méthoxyéthyle, 1,2-dihydroxyéthyle, 1-hydroxy-2-aminoéthyle, 2-hydroxy-1-aminoéthyle, phényle.

Selon un mode de réalisation particulier, R3 est choisi parmi l'atome d'hydrogène, un radical alkyle ou un radical phényle, ces radicaux étant éventuellement substitués par un hydroxy, alcoxy ou amino. De préférence, R3 représentent un atome d'hydrogène, un radical méthyle, 2-hydroxyéthyle, 2-carboxyéthyle, 1,2-dihydroxyéthyle ou phényle.

A titre d'exemple pour R4, on peut citer un radical méthyle, éthyle, isopropyle, méthoxyméthyle, hydroxyméthyle, 1-carboxyméthyle, 2-carboxyéthyle, 2-hydroxyéthyle, 3-hydroxypropyle, amino, méthylamino, diméthylamino, 2-hydroxyéthylamino, carboxyméthylamino, bis(2-hydroxyéthyl)amino, aniline, phényle, 4-aminophényle, 4-hydroxyphényle, 4-diméthylaminophényle, 4-méthoxyphényle, thiazolyle, pyrazolyle, pyridyle, aminothiazolyle, aminopyrazolyle, aminopyridyle.

Selon un mode de réalisation particulier, R₄ représente
- un radical OR₁₀;
- un radical NR₁₅R₁₆ ;
- un radical phényle éventuellement substitué par un ou plusieurs radicaux hydroxy, alcoxy en C₁-C₂, amino, (di-)méthylamino, 2-hydroxyéthylamino, bis-(2-hydroxyéthyl)amino, chlore ;
- un radical hétéroaromatique à 5 ou 6 chaînons éventuellement substitué choisi parmi les radicaux pyridyle, pyrimidinyle, imidazolyle, pyrazolyle, thiényle, thiazolyle
- un radical alkyle en C₁-C₆ linéaire ou ramifié éventuellement substitué par un ou plusieurs radicaux hydroxy, alcoxy, amino, carboxy, mono ou di alkyl(C₁-C₄)carboxamido, alkyl(C₁-C₄)sulfonamido, NR₂₅R₂₆
où R₁₅, R₁₆, R₂₅ et R₂₆ représentent préférentiellement indépendamment l'un de l'autre un atome d'hydrogène, un radical phényle éventuellement substitué, un radical pyrazolyle, thiazolyle, triazolyle, pyridyle, pyrimidinyle, un radical alkyle en C₁-C₂ éventuellement substitué par un radical hydroxy ou alcoxy en C₁-C₂.

Selon un mode préféré, R4 est choisi parmi un radical alkyle, (di)alkylamino éventuellement cyclique, aminophényle, aminopyrazolyle, aminopyridyle ou aminothiazolyle.

R4 est de préférence choisi parmi les radicaux méthyle, hydroxyméthyle, 2-hydroxyéthyle, amino, 2-hydroxyéthylamino, diméthylamino, un radical phényle, un radical aniline, un radical aminopyrazolyle, aminothiazolyle, aminotriazolyle, aminopyridyle.

Dans la formule(I) ci dessus, X représente de préférence un atome d'hydrogène, un atome de chlore, un radical alcoxy, par exemple méthoxy.

Selon un mode de réalisation particulièrement préféré, le composé de formule(I) correspond à la formule 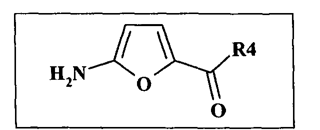 dans laquelle R4 est tel que défini précédemment.

Parmi les composés de formule (I) conformes à l'invention, on peut citer à titre d'exemple les composés suivants :
2-amino-5-carboxamidofurane
2-méthylamino-5-carboxamidofurane
2-(2-hydroxéthyl)amino-5-carboxamidofurane
2-(pyrrolidin-1-yl)-5-carboxamidofurane
2-amino-5-[N,N-diméthyl-carboxamido]-furane
2-méthylamino-5-[N,N-diméthyl-carboxamido]-furane
2-(2-hydroxéthyl)amino-5-[N,N-diméthyl-carboxamido]-furane
2-(pyrrolidin-1-yl)-5-[N,N-diméthyl-carboxamido]-furane
2-amino-5-[N-phényl-carboxamido]-furane
2-méthylamino-5-[N-phényl-carboxamido]-furane
2-(2-hydroxéthyl)amino-5-[N-phényl-carboxamido]-furane
2-(pyrrolidin-1-yl)-5-[N-phényl-carboxamido]-furane
2-amino-5-[N-(pyrazol-5-yl)-carboxamido]-furane
2-méthylamino-5-[N-(pyrazol-5-yl)-carboxamido]-furane
2-(2-hydroxéthyl)amino-5-[N-(pyrazol-5-yl)-carboxamido]-furane
2-(pyrrolidin-1-yl)-5-[N-(pyrazol-5-yl)-carboxamido]-furane
2-amino-5-[N-(1-méthylpyrazol-5-yl)-carboxamido]-furane
2-méthylamino-5-[N-(1-méthyl pyrazol-5-yl)-carboxamido]-furane
2-(2-hydroxéthyl)amino-5-[N-(1-méthyl pyrazol-5-yl)-carboxamido]-furane
2-(pyrrolidin-1-yl)- 5-[N-(1-méthyl pyrazol-5-yl)-carboxamido]-furane
2-amino-5-[N-(thiazol-2-yl)-carboxamido]-furane
2-méthylamino-5-[N-(thiazol-2-yl)-carboxamido]-furane
2-(2-hydroxéthyl)amino-5-[N-(thiazol-2-yl)-carboxamido]-(furane
2-(pyrrolidin-1-yl)- 5-[N-(thiazol-2-yl)-carboxamido]-furane

2-amino-5-[N-(triazol-3-yl)-carboxamido]-furane
2-méthylamino-5-[N-(triazol-3-yl)-carboxamido]-furane
2-(2-hydroxéthyl)amino-5-[N-(triazol-3-yl)-carboxamido]-furane
2-(pyrrolidin-1-yl)-5-[N-(triazol-3-yl)-carboxamido]-furane
2-amino-5-[N-(pyrid-2-yl)-carboxamido]-furane
2-méthylamino-5-[N-(pyrid-2-yl)-carboxamido]-furane
2-(2-hydroxéthyl)amino-5-[N-(pyrid-2-yl)-carboxamido]-furane
2-(pyrrolidin-1-yl)-5-[N-(pyrid-2-yl)-carboxamido]-furane
2-amino-5-[N-(piperidin-1-yl)-carboxamido]-furane
2-méthylamino-5-[N-(piperidin-1-yl)-carboxamido]-furane
2-(2-hydroxéthyl)amino-5-[N-(piperidin-1-yl)-carboxamido]-furane
2-(pyrrolidin-1-yl)-5-[N-(piperidin-1-yl)-carboxamido]-furane

2-amino-3-chloro-5-carboxamidofurane
2-méthylamino-3-chloro-5-carboxamidofurane
2-(2-hydroxéthyl)amino-3-chloro-5-carboxamidofurane
2-(pyrrolidin-1-yl)-3-chloro-5-carboxamidofurane
2-amino-3-chloro-5-[N,N-diméthyl-carboxamido]-furane
2-méthylamino-3-chloro-5-[N,N-diméthyl-carboxamido]-furane
2-(2-hydroxéthyl)amino-3-chloro-5-[N, N-diméthyl-carboxamido]-furane
2-(pyrrolidin-1-yl)-3-chloro-5-[N,N-diméthyl-carboxamido]-furane
2-amino-3-chloro-5-[N-phényl-carboxamido]-furane
2-méthylamino-3-chloro-5-[N-phényl-carboxamido]-furane
2-(2-hydroxéthyl)amino-3-chloro-5-[N-phényl-carboxamido]-furane
2-(pyrrolidin-1-yl)-3-chloro-5-[N-phényl-carboxamido]-furane
2-amino-3-chloro-5-[N-(pyrazol-5-yl)-carboxamido]-furane
2-méthylamino-3-chloro-5-[N-(pyrazol-5-yl)-carboxamido]-furane
2-(2-hydroxéthyl)amino-3-chloro-5-[N-(pyrazol-5-yl)-carboxamido]-furane
2-(pyrrolidin-1-yl)-3-chloro-5-[N-(pyrazol-5-yl)-carboxamido]-furane
2-amino-3-chloro-5-[N-(thiazol-2-yl)-carboxamido]-furane
2-méthylamino-3-chloro-5-[N-(thiazol-2-yl)-carboxamido]-furane
2-(2-hydroxéthyl)amino-3-chloro-5-[N-(thiazol-2-yl)-carboxamido]-furane
2-(pyrrolidin-1-yl)-3-chloro-5-[N-(thiazol-2-yl)-carboxamido]-furane
ainsi que leurs sels d'addition avec un acide ou une base.

Le composé 2-amino-5-(N-thiazolyl)carboxamidofurane et ses sels d'addition sont particulièrement préférés.

La composition de teinture par oxydation de la présente invention comprend une ou plusieurs bases d'oxydation classiquement utilisées en teinture d'oxydation. A titre d'exemple, ces bases d'oxydation sont choisies parmi les bases d'oxydation, par exemple les paraphénylènediamines, les bis-phénylalkylènediamines, les para-aminophénols, les ortho-aminophénols, les bases hétérocycliques et leurs sels d'addition avec un acide ou une base.

Parmi les paraphénylènediamines, on peut citer à titre d'exemple, la paraphénylènediamine, la paratoluylènediamine, la 2-chloro paraphénylènediamine, la 2,3-diméthyl paraphénylènediamine, la 2,6-diméthyl paraphénylènediamine, la 2,6-diéthyl paraphénylènediamine, la 2,5-diméthyl paraphénylènediamine, la N,N-diméthyl paraphénylènediamine, la N,N-diéthyl paraphénylènediamine, la N,N-dipropyl paraphénylènediamine, la 4-amino N,N-diéthyl 3-méthyl aniline, la N,N-bis-(β-hydroxyéthyl) paraphénylènediamine, la 4-N,N-bis-(β-hydroxyéthyl)amino 2-méthyl aniline, la 4-N,N-bis-(β-hydroxyéthyl)amino 2-chloro aniline, la 2-β-hydroxyéthyl paraphénylènediamine, la 2-fluoro paraphénylènediamine, la 2-isopropyl paraphénylènediamine, la N-(β-hydroxypropyl) paraphénylènediamine, la 2-hydroxyméthyl paraphénylènediamine, la N,N-diméthyl 3-méthyl paraphénylènediamine, la N,N-(éthyl, β-hydroxyéthyl) paraphénylènediamine, la N-(β,γ-dihydroxypropyl) paraphénylènediamine, la N-(4'-aminophényl) paraphénylènediamine, la N-phényl paraphénylènediamine, la 2-β-hydroxyéthyloxy paraphénylènediamine, la 2-β-acétylaminoéthyloxy paraphénylènediamine, la N-(β-méthoxyéthyl) paraphénylènediamine, la 4-aminophénylpyrrolidine, la 2-thiényl paraphénylènediamine, le 2-β hydroxyéthylamino 5-amino toluène et leurs sels d'addition avec un acide ou une base.

Parmi les paraphénylènediamines citées ci-dessus, la paraphénylènediamine, la paratoluylènediamine, la 2-isopropyl paraphénylènediamine, la 2-β-hydroxyéthyl paraphénylènediamine, la 2-β-hydroxyéthyloxy paraphénylènediamine, la 2,6-diméthyl paraphénylènediamine, la 2,6-diéthyl paraphénylènediamine, la 2,3-diméthyl paraphénylènediamine, la N,N-bis-(β-hydroxyéthyl) paraphénylènediamine, la 2-chloro paraphénylènediamine, la 2-β-acétylaminoéthyloxy paraphénylènediamine, et leurs sels d'addition avec un acide ou une base sont particulièrement préférées.

Parmi les bis-phénylalkylènediamines, on peut citer à titre d'exemple, le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthyl-aminophényl) tétraméthylènediamine, la N,N'-bis-(éthyl) N,N'-bis-(4'-amino, 3'-méthylphényl) éthylènediamine, le 1,8-bis-(2,5-diamino phénoxy)-3,6-dioxaoctane, et leurs sels d'addition avec un acide ou une base.

Parmi les para-aminophénols, on peut citer à titre d'exemple, le para-aminophénol, le 4-amino 3-méthyl phénol, le 4-amino 3-fluoro phénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, le 4-amino 2-fluoro phénol, et leurs sels d'addition avec un acide ou une base.

Parmi les ortho-aminophénols, on peut citer à titre d'exemple, le 2-amino phénol, le 2-amino 5-méthyl phénol, le 2-amino 6-méthyl phénol, le 5-acétamido 2-amino phénol, et leurs sels d'addition avec un acide ou une base.

Parmi les bases hétérocycliques, on peut citer à titre d'exemple, les dérivés pyridiniques, les dérivés pyrimidiniques et les dérivés pyrazoliques.

Parmi les dérivés pyridiniques, on peut citer les composés décrits par exemple dans les brevets GB 1 026 978 et GB 1 153 196, comme la 2,5-diamino pyridine, la 2-(4-méthoxyphényl)amino 3-amino pyridine, la 2,3-diamino 6-méthoxy pyridine, la 2-(β-méthoxyéthyl)amino 3-amino 6-méthoxy pyridine, la 3,4-diamino pyridine, et leurs sels d'addition avec un acide ou une base.

Parmi les dérivés pyrimidiniques, on peut citer les composés décrits par exemple dans les brevets DE 2 359 399 ; JP 88-169 571 ; JP 05 163 124 ; EP 0 770 375 ou demande de brevet WO 96/15765 comme la 2,4,5,6-tétra-aminopyrimidine, la 4-hydroxy 2,5,6-triaminopyrimidine, la 2-hydroxy 4,5,6-triaminopyrimidine, la 2,4-dihydroxy 5,6-diaminopyrimidine, la 2,5,6-triaminopyrimidine, et les dérivés pyrazolo-pyrimidiniques tels ceux mentionnés dans la demande de brevet FR-A-2 750 048 et parmi lesquels on peut citer la pyrazolo-[1,5-a]-pyrimidine-3,7-diamine ; la 2,5-diméthyl pyrazolo-[1,5-a]-pyrimidine-3,7-diamine ; la pyrazolo-[1,5-a]-pyrimidine-3,5-diamine ; la 2,7-diméthyl pyrazolo-[1,5-a]-pyrimidine-3,5-diamine ; le 3-amino pyrazolo-[1,5-a]-pyrimidin-7-ol ; le 3-amino pyrazolo-[1,5-a]-pyrimidin-5-ol ; le 2-(3-amino pyrazolo-[1,5-a]-pyrimidin-7-ylamino)-éthanol, le 2-(7-amino pyrazolo-[1,5-a]-pyrimidin-3-ylamino)-éthanol, le 2-[(3-amino-pyrazolo[1,5-a]pyrimidin-7-yl)-(2-hydroxy-éthyl)-amino]-éthanol, le 2-[(7-amino-pyrazolo[1,5-a]pyrimidin-3-yl)-(2-hydroxy-éthyl)-amino]-éthanol, la 5,6-diméthyl pyrazolo-[1,5-a]-pyrimidine-3,7-diamine, la 2,6-diméthyl pyrazolo-[1,5-a]-pyrimidine-3,7-diamine, la 2, 5, N 7, N 7-tetraméthyl pyrazolo-[1,5-a]-pyrimidine-3,7-diamine, la 3-amino-5-méthyl-7-imidazolylpropylamino pyrazolo-[1,5-a]-pyrimidine et leurs sels d'addition avec un acide et leurs formes tautomères, lorsqu'il existe un équilibre tautomérique.

Parmi les dérivés pyrazoliques, on peut citer les composés décrits dans les brevets DE 3 843 892, DE 4 133 957 et demandes de brevet WO 94/08969, WO 94/08970, FR-A-2 733 749 et DE 195 43 988 comme le 4,5-diamino 1-méthyl pyrazole, le 4,5-diamino 1-(β-hydroxyéthyl) pyrazole, le 3,4-diamino pyrazole, le 4,5-diamino 1-(4'-chlorobenzyl) pyrazole, le 4,5-diamino 1,3-diméthyl pyrazole, le 4,5-diamino 3-méthyl 1-phényl pyrazole, le 4,5-diamino 1-méthyl 3-phényl pyrazole, le 4-amino 1,3-diméthyl 5-hydrazino pyrazole, le 1-benzyl 4,5-diamino 3-méthyl pyrazole, le 4,5-diamino 3-tert-butyl 1-méthyl pyrazole, le 4,5-diamino 1-tert-butyl 3-méthyl pyrazole, le 4,5-diamino 1-(β-hydroxyéthyl) 3-méthyl pyrazole, le 4,5-diamino 1-éthyl 3-méthyl pyrazole, le 4,5-diamino 1-éthyl 3-(4'-méthoxyphényl) pyrazole, le 4,5-diamino 1-éthyl 3-hydroxyméthyl pyrazole, le 4,5-diamino 3-hydroxyméthyl 1-méthyl pyrazole, le 4,5-diamino 3-hydroxyméthyl 1-isopropyl pyrazole, le 4,5-diamino 3-méthyl 1-isopropyl pyrazole, le 4-amino 5-(2'-aminoéthyl)amino 1,3-diméthyl pyrazole, le 3,4,5-triamino pyrazole, le 1-méthyl 3,4,5-triamino pyrazole, le 3,5-diamino 1-méthyl 4-méthylamino pyrazole, le 3,5-diamino 4-(β-hydroxyéthyl)amino 1-méthyl pyrazole, et leurs sels d'addition avec un acide ou une base.

La ou les bases d'oxydation sont en général chacune présentes en quantité comprise entre 0,001 à 10 % en poids environ du poids total de la composition tinctoriale, de préférence entre 0,005 et 6 %.

La composition selon l'invention peut contenir un ou plusieurs coupleurs additionnels conventionnellement utilisés pour la teinture de fibres kératiniques. Parmi ces coupleurs additionnels, on peut notamment citer les métaphénylènediamines, les méta-aminophénols, les métadiphénols, les coupleurs naphtaléniques, les coupleurs hétérocycliques autres que ceux de formule (I) et leur sels d'addition avec un acide ou une base.

A titre d'exemple, on peut citer le 2-méthyl 5-aminophénol, le 5-N-(β-hydroxyéthyl)amino 2-méthyl phénol, le 6-chloro-2-méthyl-5-aminophénol, le 3-amino phénol, le 1,3-dihydroxy benzène, le 1,3-dihydroxy 2-méthyl benzène, le 4-chloro 1,3-dihydroxy benzène, le 2,4-diamino 1-(β-hydroxyéthyloxy) benzène, le 2-amino 4-(β-hydroxyéthylamino) 1-méthoxybenzène, le 1,3-diamino benzène, le 1,3-bis-(2,4-diaminophénoxy) propane, la 3-uréido aniline, le 3-uréido 1-diméthylamino benzène, le sésamol, le 1-β-hydroxyéthylamino-3,4-méthylènedioxybenzène, l'α-naphtol, le 2 méthyl-1-naphtol, le 6-hydroxy indole, le 4-hydroxy indole, le 4-hydroxy N-méthyl indole, la 2-amino-3-hydroxy pyridine, la 6- hydroxy benzomorpholine la 3,5-diamino-2,6-diméthoxypyridine, le 1-N-(β-hydroxyéthyl)amino-3,4-méthylène dioxybenzène, le 2,6-bis-(ß-hydroxyéthylamino)toluène et leurs sels d'addition avec un acide ou une base.

Dans la composition de la présente invention, le ou les coupleurs sont chacun généralement présents en quantité comprise entre 0,001 et 10 % en poids environ du poids total de la composition tinctoriale, de préférence entre 0,005 et 6 %.

D'une manière générale, les sels d'addition des bases d'oxydation et des coupleurs utilisables dans le cadre de l'invention sont choisis parmi les sels d'addition avec un acide tels que les chlorhydrates, les bromhydrates, les sulfates, les citrates, les succinates, les tartrates, les lactates, les tosylates, les benzènesulfonates, les phosphates et les acétates et les sels d'addition avec une base telles que la soude, la potasse, l'ammoniaque, les amines ou les alcanolamines.

La composition tinctoriale conforme à l'invention peut en outre contenir un ou plusieurs colorants directs pouvant notamment être choisis parmi les colorants nitrés de la série benzénique, les colorants directs azoïques, les colorants directs méthiniques. Ces colorants directs peuvent être de nature non ionique, anionique ou cationique.

Le milieu approprié pour la teinture appelé aussi support de teinture est un milieu cosmétique généralement constitué par de l'eau ou par un mélange d'eau et d'au moins un solvant organique pour solubiliser les composés qui ne seraient pas suffisamment solubles dans l'eau. A titre de solvant organique, on peut par exemple citer les alcanols inférieurs en C₁-C₄, tels que l'éthanol et l'isopropanol ; les polyols et éthers de polyols comme le 2-butoxyéthanol, le propylèneglycol, le monométhyléther de propylèneglycol, le monoéthyléther et le monométhyléther du diéthylèneglycol, ainsi que les alcools aromatiques comme l'alcool benzylique ou le phénoxyéthanol, et leurs mélanges.

Les solvants sont, de préférence, présents dans des proportions de préférence comprises entre 1 et 40 % en poids environ par rapport au poids total de la composition tinctoriale, et encore plus préférentiellement entre 5 et 30 % en poids environ.

La composition tinctoriale conforme à l'invention peut également renfermer divers adjuvants utilisés classiquement dans les compositions pour la teinture des cheveux, tels que des agents tensio-actifs anioniques, cationiques, non-ioniques, amphotères, zwittérioniques ou leurs mélanges, des polymères anioniques, cationiques, non-ioniques, amphotères, zwittérioniques ou leurs mélanges, des agents épaississants minéraux ou organiques, et en particulier les épaississants associatifs polymères anioniques, cationiques, non ioniques et amphotères, des agents antioxydants, des agents de pénétration, des agents séquestrants, des parfums, des tampons, des agents dispersants, des agents de conditionnement tels que par exemple des silicones volatiles ou non volatiles, modifiées ou non modifiées, des agents filmogènes, des céramides, des agents conservateurs, des agents opacifiants.

Les adjuvants ci dessus sont en général présents en quantité comprise pour chacun d'eux entre 0,01 et 20 % en poids par rapport au poids de la composition.

Bien entendu, l'homme de l'art veillera à choisir ce ou ces éventuels composés complémentaires de manière telle que les propriétés avantageuses attachées intrinsèquement à la composition de teinture d'oxydation conforme à l'invention ne soient pas, ou substantiellement pas, altérées par la ou les adjonctions envisagées.

Le pH de la composition tinctoriale conforme à l'invention est généralement compris entre 3 et 12 environ, et de préférence entre 5 et 11 environ. Il peut être ajusté à la valeur désirée au moyen d'agents acidifiants ou alcalinisants habituellement utilisés en teinture des fibres kératiniques ou bien encore à l'aide de systèmes tampons classiques.

Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule (II) suivante : 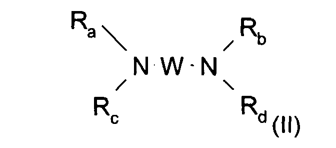 dans laquelle W est un reste propylène éventuellement substitué par un groupement hydroxyle ou un radical alkyle en C₁-C₄ ; Rₐ, R_{b}, R_{c} et R_{d}, identiques ou différents, représentent un atome d'hydrogène, un radical alkyle en C₁-C₄ ou hydroxyalkyle en C₁-C₄.

La composition tinctoriale selon l'invention peut se présenter sous des formes diverses, telles que sous forme de liquides, de crèmes, de gels, ou sous toute autre forme appropriée pour réaliser une teinture des fibres kératiniques, et notamment des cheveux humains.

Le procédé de la présente invention est un procédé dans lequel on applique sur les fibres la composition selon la présente invention telle que définie précédemment, en présence d'un agent oxydant pendant un temps suffisant pour obtenir la coloration désirée. La couleur peut être révélée à pH acide, neutre ou alcalin et l'agent oxydant peut être ajouté à la composition de l'invention juste au moment de l'emploi ou il peut être mis en oeuvre à partir d'une composition oxydante le contenant, appliquée simultanément ou séquentiellement à la composition de l'invention.

Selon un mode de réalisation particulier, la composition selon la présente invention est mélangée, de préférence au moment de l'emploi, à une composition contenant, dans un milieu approprié pour la teinture, au moins un agent oxydant, cet agent oxydant étant présent en une quantité suffisante pour développer une coloration. Le mélange obtenu est ensuite appliqué sur les fibres kératiniques. Après un temps de pose de 3 à 50 minutes environ, de préférence 5 à 30 minutes environ, les fibres kératiniques sont rincées, lavées au shampooing, rincées à nouveau puis séchées.

Les agents oxydants classiquement utilisés pour la teinture d'oxydation des fibres kératiniques sont par exemple le peroxyde d'hydrogène, le peroxyde d'urée, les bromates de métaux alcalins, les persels tels que les perborates et persulfates, les peracides et les enzymes oxydases parmi lesquelles on peut citer les peroxydases, les oxydo-réductases à 2 électrons telles que les uricases et les oxygénases à 4 électrons comme les laccases. Le peroxyde d'hydrogène est particulièrement préféré.

La composition oxydante peut également renfermer divers adjuvants utilisés classiquement dans les compositions pour la teinture des cheveux et tels que définis précédemment.

Le pH de la composition oxydante renfermant l'agent oxydant est tel qu'après mélange avec la composition tinctoriale, le pH de la composition résultante appliquée sur les fibres kératiniques varie de préférence entre 3 et 12 environ, et encore plus préférentiellement entre 5 et 11. Il peut être ajusté à la valeur désirée au moyen d'agents acidifiants ou alcalinisants habituellement utilisés en teinture des fibres kératiniques et tels que définis précédemment.

La composition prête à l'emploi qui est finalement appliquée sur les fibres kératiniques peut se présenter sous des formes diverses, telles que sous forme de liquides, de crèmes, de gels ou sous toute autre forme appropriée pour réaliser une teinture des fibres kératiniques, et notamment des cheveux humains.

L'invention a aussi pour objet un dispositif à plusieurs compartiments ou "kit" de teinture dans lequel un premier compartiment renferme la composition tinctoriale de la présente invention définie ci-dessus et un deuxième compartiment renferme un agent oxydant. Ce dispositif peut être équipé d'un moyen permettant de délivrer sur les cheveux le mélange souhaité, tel que les dispositifs décrits dans le brevet FR-2 586 913 au nom de la demanderesse.

A partir de ce dispositif, il est possible de teindre les fibres kératiniques à partir d'un procédé qui comprend le mélange d'une composition tinctoriale comprenant au moins une base d'oxydation de formule (I) avec un agent oxydant, et l'application du mélange obtenu sur les fibres kératiniques pendant un temps suffisant pour développer la coloration désirée.

Les composés de formule (I) peuvent être obtenus selon les méthodes de synthèse connues dans le domaine de la synthèse d'hétérocycle. On peut se référer par exemple aux publications Khim.Geterosilk.Soedin, 1969, 17 ; Liebigs Ann.Chem., 1986, 625 ; Tetrahedron Letter, 1987, 28(36), 4123 ; J.Org.Chem., 1978, 43, 4303; Helv.Chim.Acta., 1977, 60, 208; Tetrahedron Letter, 2000, 41(34), 6531 ; Chemicke Zvesti, 1969, 23(2), 139 ; J.Org.Chem. 1998, 63(12), 3986 ; J.Org.Chem. 59, 5034, 1994 ; Org.Synth., 75, 61, 1997 ; EP219852 ; JP61022075.

Selon un mode de réalisation particulier, certains des composés de formule(I) peuvent être obtenus selon le schéma suivant :

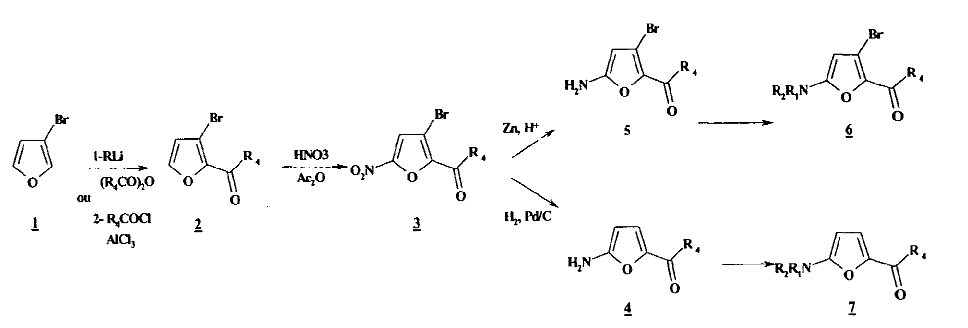

Les dérivés furaniques **2** acylés en position 2 sont obtenus par réaction du 3-bromofurane avec un organolithien, tel que le butyllithium, lesec-butyllithium, le ter-butyllithium, à des températures comprises entre -78°C et 20°C, et un anhydride du type (R₄CO)₂O. Une autre méthode consiste à faire réagir le 3-bromofurane avec le chlorure d'acide R₄COCI en présence de trichlorure d'aluminium pour conduire aux dérivés **2**. L'introduction d'un groupe nitro en position 5 du dérivé **2** est réalisée par réaction classique d'acide nitrique dans un solvant protique acide, tel que l'acide acétique, l'acide sulfurique, l'acide chlorhydrique, à des températures comprises entre 0°C et 30°C, conduisant ainsi aux composés 2-nitro-5-carbonyl-3-bromofurane **3**. Ceux-ci sont ensuite soumis à une réaction d'hydrogénation par catalyse hétérogène telle que Pd/C, Pd(II)/C, Ni/Ra, etc... pour conduire aux dérivés 2-amino-5-carbonyl-furane **4** ou encore à une réaction de réduction par un métal tel que par du zinc, fer, étain, etc... pour conduire aux dérivés 2-amino-5-carbonyl-3-bromofurane **5** ( voir Advanced Organic Chemistry, 3^{ème} édition, J. March, Willey Interscience et Réduction in organic Chemistry, M. Hudlicky, Ellis Horwood series chemical Science). La fonctionalisation de l'amine primaire en position 2 en amines secondaires et tertiaires NR₁R₂ pour obtenir les dérivés souhaités **6** et **7** ( éventuellement formant un cycle) est réalisée selon les méthodes classiques de synthèse organique ( halogénure d'alkyle, O-sulfonate d'alkyle, trialkylammonium d'alkyle, amination réductrice, etc...voir par exemple *Advanced Organic Chemistry, 3*^{*ème*} *édition, J. March, Willey Interscience.*

Selon un autre mode de réalisation particulier, certains des composés de formule(I) peuvent être obtenus selon le schéma suivant :

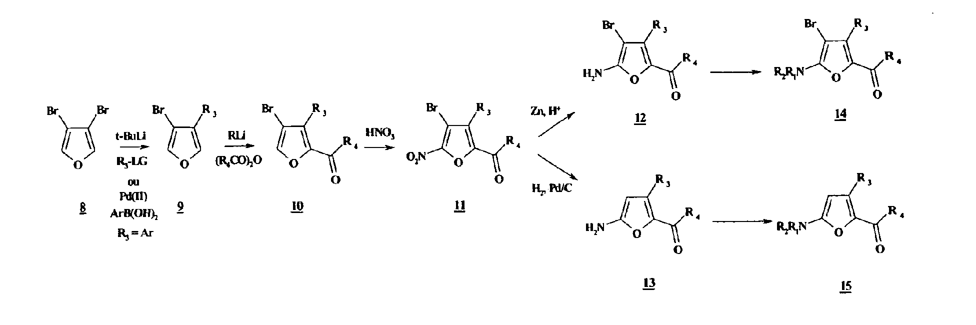

L'introduction du groupe R₃ en position 4 est réalisée par réaction du dérivé dibromé **8** et de t-BuLi dans un solvant aprotique, tel que l'éther diéthylique ou le THF, et à des températures comprises entre -110°C et 20°C, conduisant au composé lithié en position 4, celui réagissant ainsi avec les électrophiles du type R₃-LG, LG étant un groupe nucléofuge, tel que par exemple un halogénure ou un O-sulfonate d'alkyle. Lorsque R3 est un dérivé benzénique, le dibromofurane **3** est mis en réaction en présence de Pd(II), tel que l'acétate de palladium, et d'acide arylboronique. Les dérivés 3-bromofurane **9** fonctionnalisés en position 4 par le groupe R₃ réagissent avec un organolithien, tel que le butyllithium, les sec-butyllithiul, le ter-butyllithium, à des températures comprises entre -78°C et 20°C, et un anhydride du type (R₄CO)₂O pour conduire aux dérivés **10**. Une autre méthode consiste à faire réagir le 3-bromofurane **9** avec le chlorure d'acide R₄COCI en présence de trichlorure d'aluminium pour conduire aux dérivés **10**. L'introduction d'un groupe nitro en position 5 du dérivé **10** est réalisée par réaction classique d'acide nitrique dans un solvant protique acide, tel que l'acide acétique, l'acide sulfurique, l'acide chlorhydrique, à des températures comprises entre 0°C et 30°C, conduisant ainsi aux composés 2-nitro-5-carbonyl-3-bromofurane **11.** Ceux-ci sont ensuite soumis à une réaction d'hydrogénation par catalyse hétérogène telle que Pd/C, Pd(II)/C, Ni/Ra, etc... pour conduire aux dérivés 2-amino-5-carbonyl-furane **13** substitué en position 4 par le groupe R₃ ou encore à une réaction de réduction par un métal tel que par du zinc, fer, étain, etc... pour conduire aux dérivés 2-amino-5-carbonyl-3-bromofurane **12** substitués an position 4 par le groupe R_{3_}( voir Advanced Organic Chemistry, 3^{ème} édition, J. March, Willey Interscience et Réduction in organic Chemistry, M. Hudlicky, Ellis Horwood series chemical Science). La fonctionalisation de l'amine primaire en position 2 en amines secondaires et tertiaires NR₁R₂ pour obtenir les dérivés souhaités **14** et **15** ( éventuellement formant un cycle) est réalisée selon les méthodes classiques de synthèse organique ( halogénure d'alkyle, O-sulfonate d'alkyle, trialkylammonium d'alkyle, amination réductrice, etc...voir par exemple *Advanced Organic Chemistry, 3*^{*ème*} *édition, J. March , Willey Interscience.*

Selon un mode de réalisation différent, certains des composés de formule (I) peuvent être obtenus selon le schéma suivant :

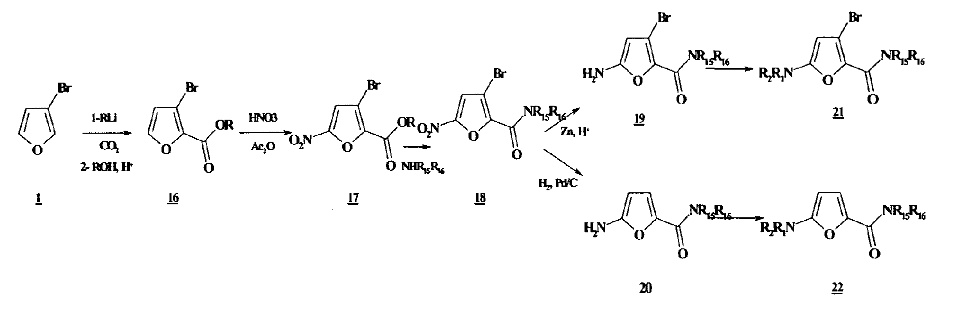

Les dérivés 5-carboxamido **21** et **22** sont obtenus selon la méthode de synthèse décrite ci-dessous. La réaction du 2-lithié-3-bromofurane avec du CO₂ conduit au composés **16** après estérification en présence d'un alcool ROH et d'acide protique en quantité catalytique. La réaction de nitration dans les conditions classiques telles que décrites ci-dessus conduit aux dérivés furaniques **17**. La réaction d'amine NHR₁₅R₁₆ sur l'ester **17** permet d'obtenir les dérivés 5-carboxamido-2-nitro-3-bromofuranique **18**. Les réactions de réduction et fonctionnalisation des amines correspondantes selon les méthodes décrites ci-dessus conduisent aux composés **19** à **22**.

La présente invention concerne de plus les composés nouveaux de formule (I) dans laquelle R1, R2, R3 et X sont tels que définis précédemment et R4 représente
- un radical phényle éventuellement substitué
- un radical hétéroaromatique éventuellement substitué choisi parmi un furyle, thiényle, oxazole, imidazole, thiazole, isoxazole, triazole, thiadiazole, pyridyle, pyrimidyle, pyrazinyle, pyridazinyle ;
- un radical NR₁₅R₁₆ ;
- un radical alkyle en C₂-C₈ linéaire ou ramifié pouvant être substitué par un ou plusieurs radicaux hydroxy, alcoxy OR₁₀, carboxy, carboxamido, alkylsulfonyle, , alklsulfoxyde, alkylsulfonamido, NR₂₅R₂₆ ,
- R₁₅, R₁₆, R₂₅ et R₂₆ représentent indépendamment l'un de l'autre un atome d'hydrogène, un radical phényle éventuellement substitué, un radical hétéroaromatique éventuellement substitué, un radical alkyle en C₁-C₆ éventuellement substitué par un ou plusieurs radicaux choisis dans le groupe constitué par un hydroxy, un alcoxy en C₁-C₂, un alkyl(C₁-C₄)sulfonamido, un carboxy, un carboxamido, un alkyl(C₁-C₄)sulfonyle, un alkyl(C₁-C₄)sulfoxyde, un amino, un (di)alkylamino, un (poly)hydroxyalkylamino en C₂-C₄,
   - R₁₀ représente un radical alkyle en C₁-C₄ éventuellement substitué par un ou plusieurs radicaux choisis parmi les radicaux hydroxy, alcoxy en C₁-C₂, amino, (di)(C₁-C₄)alkylamino, (poly)hydroxyalkylamino en C₂-C₄.

Les exemples qui suivent servent à illustrer l'invention sans toutefois présenter un caractère limitatif.

### EXEMPLES

### Exemple de synthèse

A 10 ml d'une solution hydroalcolique (80:20 éthanol:eau), contenant 10% de chlorure d'ammonium et 0,5 g de zinc sous forme de poudre sont ajoutés 1 mmole de dérivé 2-nitro-5-[N-(thiazol-2-yl)carboxamido]-furane à une température de 80°C. Après consommation totale du produit de départ nitré, le mélange réactionnel est concentré jusqu'à précipitation d'un solide qui est essoré, lavé par de l'éther diisopropylique et séché sous vide jusqu'à poids constant.
On obtient ainsi le 2-amino-5-[(N-thiazol-2-yl)carboxamido]-furane :

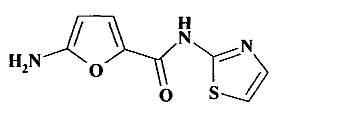

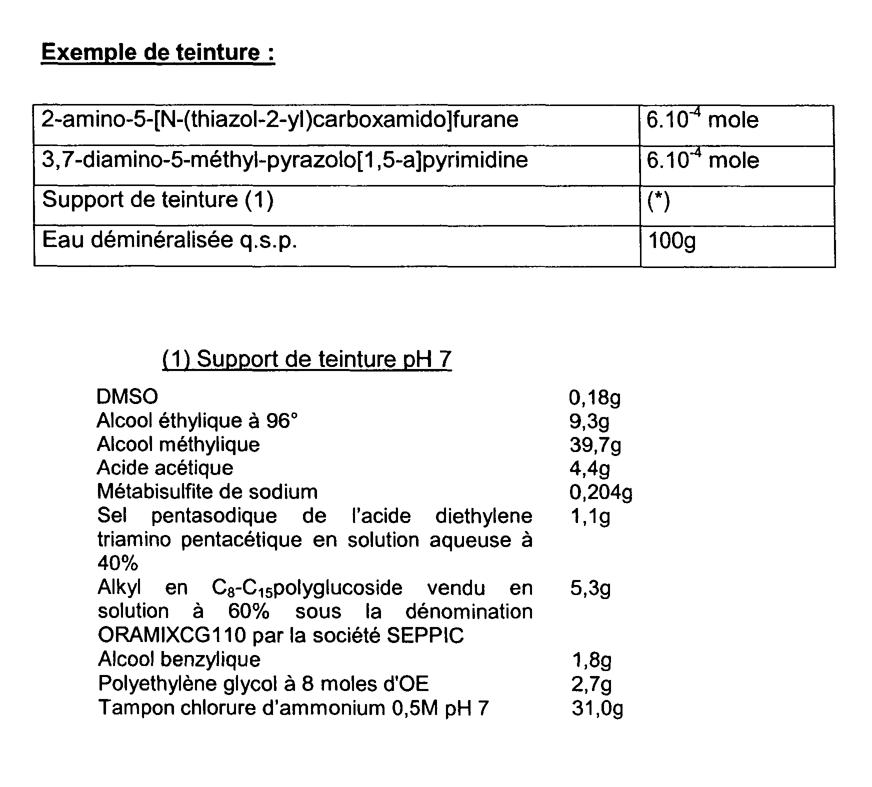

Au moment de l'emploi, chaque composition est mélangée avec un poids égal d'eau oxygénée à 20 volumes (6% en poids). On obtient un pH final de 7.

Chaque mélange obtenu est appliqué sur des mèches de cheveux gris à 90 % de blancs. Après 30 min de pose, les mèches sont rinçées, lavées avec un shampooing standard, rinçées à nouveau puis séchées.

La nuance obtenue est orange.

## Revendications

1. Composition tinctoriale comprenant, dans un milieu approprié pour la teinture, au moins une base d'oxydation et au moins un coupleur de type furane de formule (I) suivante ainsi que ses sels d'addition avec un acide ou une base : 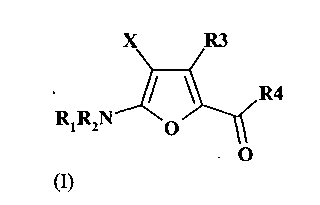 dans laquelle :
• R₁, R₂ représentent, identiques ou différents,
- un atome d'hydrogène ;
- un radical phényle éventuellement substitué par un ou plusieurs radicaux choisis parmi les halogènes, les radicaux hydroxy, alcoxy en C₁-C₄, carboxy, mono ou di alkyl(C₁-C₄)carboxamido, alkyl(C₁-C₄)sulfonyle, sulfonique, alkylsulfoxyde, alkylsulfonamido, NR₁₃R₁₄, alkyle en C₁-C₄ éventuellement substitué parmi un ou plusieurs radicaux hydroxy, alcoxy en C₁-C₄, carboxy, mono ou di alkyl(C₁-C₄)carboxamido, alkyl(C₁-C₄)sulfonyle, sulfonique, alkylsulfoxyde, alkylsulfonamido ou NR₁₃R₁₄ ;
- un radical alkyle en C₁-C₈ linéaire ou ramifié, éventuellement substitué par un ou plusieurs radicaux hydroxy, alcoxy en C₁-C₄, carboxy, mono ou di alkyl(C₁-C₄)carboxamido, alkyl(C₁-C₄)sulfonyle, sulfonique, alkylsulfoxyde, alkylsulfonamido ou NR₁₃R₁₄,
• R₁ et R₂ ensemble peuvent former avec l'atome d'azote avec lequel ils sont attachés un hétérocycle comportant 5 à 8 chaînons dont un ou plusieurs atomes de carbone du cycle carboné peuvent être remplacés par un atome d'oxygène, d'azote, de soufre ou par un groupement SO₂ ; les atomes de carbone dudit cycle peuvent être substitués par un radical R₅ ; le cycle ne comportant pas de liaison peroxyde, ni de radicaux diazo ou nitroso ;
- R₅ représente :
- un atome d'halogène ;
- un radical alkyle en C₁-C₄ éventuellement substitué par un ou plusieurs radicaux hydroxy, carboxy, alcoxycarbonyle en C₁-C₄, alcoxy en C₁-C₄, ou NR₆R₇ ;
- un radical carboxy ;
- un radical mono ou di alkylcarboxamido ;
- un radical alkyl(C1-C4)sulfonyle ;
- un radical alkylsulfonamido
- un radical hydroxy ;
- un radical alcoxy en C₁-C₄ ;
- un radical hydroxyalcoxy en C₂-C₄ ;
- un radical aminosulfonyle;
- un radical thioéther en C₁-C₄ ;
- un radical alkyl(C₁-C₄)sulfoxyde ;
- un radical alkyl(C1-C4)sulfonyle ;
- un radical NR₈R₉ ;
• R₃ représente
- un atome d'hydrogène ;
- un radical alkyl(C₁-C₄)sulfonyle;
- un radical alkyle en C₁-C₈ linéaire ou ramifié, éventuellement substitué par un ou plusieurs radicaux hydroxy, OR₁₀, carboxy, alcoxycarbonyle en C₁-C₄, mono ou di alkyl(C₁-C₄)carboxamido, alkyl(C₁-C₄)sulfonyle, sulfonique, alkyl(C₁-C₄)sulfoxyde, alkylsulfonamido (alkyl(C₁-C₄)SO₂NH-) ou NR₁₁R₁₂ ;
- un radical phényle ou un hétérocycle aromatique à 5 ou 6 chaînons, ces radicaux étant éventuellement substitués par un ou plusieurs radicaux choisis parmi les halogènes, les radicaux hydroxy, alcoxy en C₁-C₄, carboxy, alcoxycarbonyle en C₁-C₄, mono ou di alkyl(C₁-C₄)carboxamido, alkyl(C₁-C₄)sulfonyle, sulfonique, alkylsulfoxyde, thioéther en C₁-C₄, alkylsulfonamido, NR₁₃R₁₄, alkyle en C₁-C₄ éventuellement substitué par un ou plusieurs radicaux hydroxy, alcoxy en C₁-C₄, carboxy, mono ou di alkyl(C₁-C₄)carboxamido, alkyl(C₁-C₄)sulfonyle , sulfonique, alkylsulfoxyde, alkylsulfonamido ou NR₁₃R₁₄ ;
- R₁₀ représente un radical alkyle en C₁-C₄ éventuellement substitué par un ou plusieurs radicaux hydroxy, alcoxy en C₁-C₂, amino, mono ou di alkyl (C₁-C₄)amino, (poly)hydroxyalkylamino en C₂-C₄ ,
- R₆, R₇, R₈, R₉, R₁₁, R₁₂, R₁₃, R₁₄, représentent, identiques ou différents, un atome d'hydrogène, un radical sulfino, un radical alkyl(C₁-C₄)-CO, un radical mono ou di alkyl(C₁-C₄)carboxamido, un radical alkyle en C₁-C₄ éventuellement substitué par un ou plusieurs radicaux hydroxy, alcoxy en C₁-C₄, alkyl(C₁-C₄)sulfonyle, alkylsulfonamido, carboxy, mono ou di alkyl(C₁-C₄)carboxamido, alkylsulfoxyde, amino, mono- ou di-(C₁-C₄)alkylamino, (poly)hydroxyalkylamino en C₂-C₄ ;
- R₈ et R₉ peuvent également former avec l'atome d'azote sur lequel ils sont attachés un cycle de 5 à 8 chaînons éventuellement substitué par un ou plusieurs radicaux choisis parmi les halogènes, les radicaux hydroxy, alcoxy en C₁-C₂ carboxy, alkylsulfonamido, NR₁₃R₁₄, alkyle en C₁-C₄ éventuellement substitué par un ou plusieurs radicaux hydroxy, alcoxy en C₁-C₄, carboxy, mono ou di alkyl(C₁-C₄)carboxamido, alkyl(C₁-C₄)sulfonyle, sulfonique, alkylsulfoxyde, alkylsulfonamido ou NR₁₃R₁₄;
• R₄ représente
- un radical phényle éventuellement substitué par un ou plusieurs radicaux choisis parmi les halogènes, les radicaux hydroxy, alcoxy en C₁-C₄, carboxy, alcoxycarbonyle en C₁-C₄, mono ou di alkyl(C₁-C₄)carboxamido, alkyl(C₁-C₄)sulfonyle, sulfonique, alkylsulfoxyde, thioéther en C₁-C₄, alkylsulfonamido, NR₁₃R₁₄, alkyle en C₁-C₄ éventuellement substitué par un ou plusieurs radicaux hydroxy, alcoxy en C₁-C₄, carboxy, mono ou di alkyl(C₁-C₄)carboxamido, alkyl(C₁-C₄)sulfonyle , sulfonique, alkylsulfoxyde, alkylsulfonamido ou NR₁₃R₁₄,
- un radical hétéroaromatique choisi parmi un radical furyle, thiényle, oxazole, imidazole, thiazole, pyrrole, isoxazole, triazole, thiadiazole, pyridyle, pyrimidyle, pyrazinyle, pyridazinyle; éventuellement substitué un ou plusieurs radicaux choisis parmi les halogènes, les radicaux hydroxy, alcoxy en C₁-C₄, carboxy, alcoxycarbonyle en C₁-C₄, mono ou di alkyl(C₁-C₄)carboxamido, alkyl(C₁-C₄)sulfonyle, sulfonique, alkylsulfoxyde, thioéther en C₁-C₄, alkylsulfonamido, NR₁₃R₁₄, alkyle en C₁-C₄ éventuellement substitué par un ou plusieurs radicaux hydroxy, alcoxy en C₁-C₄, carboxy, mono ou di alkyl(C₁-C₄)carboxamido, alkyl(C₁-C₄)sulfonyle , sulfonique, alkylsulfoxyde, alkylsulfonamido ou NR₁₃R₁₄
- un radical OR₁₀ ;
- un radical NR₁₅R₁₆ ;
- un radical OH ;
- un radical alkyle en C₁-C₈ linéaire ou ramifié pouvant être substitué par un ou plusieurs radicaux hydroxy, OR₁₀, carboxy, alcoxycarbonyle, mono ou di alkyl(C₁-C₄)carboxamido, alkylsulfonyle, sulfino, alklsulfoxyde, alkylsulfonamido, NR₂₅R₂₆,
- R₁₅, R₁₆, R₂₅ et R₂₆ représentent indépendamment l'un de l'autre un atome d'hydrogène, un radical phényle, un radical hétéroaromatique, un radical alkyle en C₁-C₆, les radicaux R₁₅, R₁₆, R₂₅ et R₂₆ étant éventuellement substitué par un ou plusieurs radicaux hydroxy, alcoxy en C₁-C₂, phényle, alkyl(C₁-C₄)sulfonamido, carboxy, mono ou di alkyl(C₁-C₄)carboxamido, alkyl(C₁-C₄)sulfonyle, alkyl(C₁-C₄)sulfoxyde, amino, mono- ou di- alkylamino, (poly)hydroxyalkylamino en C₂-C₄,
• X représente un atome d'hydrogène ; un atome d'halogène ; un radical alcoxy en C₁-C₄ ; un radical phénoxy éventuellement substitué par un ou plusieurs radicaux choisis parmi les halogènes, les radicaux hydroxy, alcoxy en C₁-C₄, carboxy, alcoxycarbonyle en C₁-C₄, mono ou di alkyl(C₁-C⁴)carboxamido, alkyl(C₁-C₄)sulfonyle, sulfonique, alkylsulfoxyde, thioéther en C₁-C₄, alkylsulfonamido, N₁₃R₁₄, alkyle en C₁-C₄ éventuellement substitué par un hydroxy, alcoxy en C1-C2, carboxy, sulfonique, amino.

2. Composition selon la revendication 1, dans lequel R₁ et R₂ représentent, identiques ou différents, un atome d'hydrogène ; un radical phényle ; un radical alkyle en C₁-C₄, linéaire ou ramifié éventuellement substitué par un ou plusieurs radicaux hydroxy, alcoxy en C₁-C₂, amino, mono ou di(C₁-C₄)alkylamino , carboxy, mono ou di alkyl(C₁-C₄)carboxamido, sulfonamido, NR₁₃R₁₄ où R₁₃ et R₁₄ représentent, identiques ou différents, un atome d'hydrogène, acyle, un radical alkyle en C₁-C₂ éventuellement substitué par un hydroxy ou alcoxy en C₁-C₂.

3. Composition selon la revendication 1 ou 2 dans lequel R₁ et R₂ représentent, identiques ou différents, un atome d'hydrogène, un radical méthyle, éthyle, (iso)propyle, 2-hydroxyéthyle, 1-hydroxyéthyle, 2-carboxyéthyle, 2-aminoéthyle, 2-(diméthylamino)éthyle, 2-(acylamino)éthyle, 2-méthoxyéthyle, 1,2-dihydroxyéthyle, 1-hydroxy-2-aminoéthyle, 2-hydroxy-1-aminoéthyle, acétyle, ou phényle.

4. Composition selon la revendication 3 dans lequel R₁ et R₂ représentent, identiques ou différents, un atome d'hydrogène, les radicaux méthyle, 2-hydroxyéthyle, 2-carboxyéthyle ou 1,2-dihydroxyéthyle.

5. Composition selon la revendication 1 dans lequel R₁ et R₂ forment avec l'atome d'azote avec lequel ils sont attachés un hétérocycle de 5 à 8 chaînons choisi parmi les pyrrolidine, pipéridine, homopipéridine, pipérazine, homopipérazine, lesdits cycles pouvant être substitués par un ou plusieurs radicaux R₅.

6. Composition selon la revendication 5 dans lequel R₁ et R₂ forment ensemble avec l'atome d'azote auquel ils sont rattachés un hétérocycle choisi parmi la pyrrolidine, la 2,5-diméthylpyrrolidine, la proline, la 3-hydroxyproline, la 4-hydroxyproline, la 2,4-dicarboxypyrrolidine, la 3-hydroxy-2-hydroxyméthylpyrrolidine, la 2-carboxamidopyrrolidine, la 3-hydroxy-2-carboxamidopyrrolidine, la 2-(diéthylcarboxamido)pyrrolidine, la 2-hydroxyméthyl pyrrolidine, la 3,4-dihydroxy-2-hydroxyméthyl pyrrolidine, la 3-hydroxypyrrolidine, la 3,4-dihydroxy pyrrolidine, la 3-amino pyrrolidine, la 3-méthylamino pyrrolidine, la 4-amino-3-hydroxy pyrrolidine, la 3-hydroxy-4-(2-hydroxyéthyl)amino-pyrrolidine, la pipéridine, la 2,6-diméthylpipéridine, la 2-carboxypipéridine, la 2-carboxamidopipéridine, la 2-hydroxyméthylpipéridine, la 3-hydroxy-2-hydroxyméthylpipéridine, 3-hydroxypipéridine, la 4-hydroxypipéridine, la 3-hydroxyméthylpipéridine, l'homopipéridine, la 2-carboxyhomopipéridine, la 2-carboxamidohomopipéridine, l'homopiperazine, la N-méthyl homopipérazine, la N β-hydroxyéthylhomopipérazine et leurs sels d'addition.

7. Composition selon la revendication 6 dans lequel R₁ et R₂ forment ensemble avec l'atome d'azote auquel ils sont rattachés un hétérocycle choisi parmi la pyrrolidine, la 3-hydroxypyrrolidine, la 3-aminopyrrolidine, la proline, la 3-hydroxyproline, la pipéridine, la 3-hydroxypipéridine, la 3-hydroxypipéridine, l'homopipéridine, l'homopipérazine, la N-méthyl homopipérazine, la N β-hydroxyéthylhomopipérazine et leurs sels d'addition.

8. Composition selon l'une quelconque des revendications 1 à 7 dans lequel R₃ est choisi parmi l'atome d'hydrogène, un radical alkyle ou un radical phényle, ces radicaux étant éventuellement substitués par un hydroxy, alcoxy ou amino.

9. Composition selon la revendication 8 dans lequel R₃ représente un atome d'hydrogène, un radical méthyle, 2-hydroxyéthyle, 2-carboxyéthyle, 1,2-dihydroxyéthyle ou phényle.

10. Composition selon l'une quelconque des revendications 1 à 9 dans lequel R₄ représente
- un radical OR₁₀;
- un radical NR₁₅R₁₆ ;
- un radical phényle éventuellement substitué par un ou plusieurs radicaux hydroxy, alcoxy en C₁-C₂, amino, (di-)méthylamino, 2-hydroxyéthylamino, bis-(2-hydroxyéthyl)amino, chlore ;
- un radical hétéroaromatique à 5 ou 6 chaînons éventuellement substitué choisi parmi les radicaux pyridyle, pyrimidinyle, imidazolyle, pyrazolyle, thiényle, thiazolyle
- un radical alkyle en C₁-C₆ linéaire ou ramifié éventuellement substitué par un ou plusieurs radicaux hydroxy, alcoxy, amino, carboxy, mono ou di alkyl(C₁-C₄)carboxamido, alkyl(C₁-C₄)sulfonamido, NR₂₅R₂₆
où R₁₅, R₁₆, R₂₅ et R₂₆ représentent préférentiellement indépendamment l'un de l'autre un atome d'hydrogène, un radical phényle éventuellement substitué, un radical pyrazolyle, thiazolyle, triazolyle, pyridyle, pyrimidinyle, un radical alkyle en C₁-C₂ éventuellement substitué par un radical hydroxy ou alcoxy en C₁-C₂.

11. Composition selon la revendication 10 dans lequel R₄ est choisi parmi un radical alkyle, (di)alkylamino éventuellement cyclique, aminophényle, aminopyrazolyle, aminopyridyle ou aminothiazolyle.

12. Composition selon la revendication 11 dans lequel R₄ est choisi parmi les radicaux méthyle, hydroxyméthyle, 2-hydroxyéthyle, amino, 2-hydroxyéthylamino, diméthylamino, un radical phényle, un radical aniline, un radical aminopyrazolyle, aminothiazolyle, aminotriazolyle, aminopyridyle.

13. Composition selon l'une quelconque des revendications 1 à 12 dans lequel X représente un atome d'hydrogène, un atome de chlore, un radical alcoxy.

14. Composition selon l'une quelconque des revendications précédentes contenant au moins un coupleur de formule 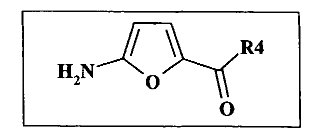 dans laquelle R4 est tel que défini précédemment aux revendications 1 à 12.

15. Composition selon l'une quelconque des revendications 1 à 14, dans laquelle le coupleur de formule (I) est choisi parmi les composés suivants :
2-amino-5-carboxamidofurane
2-méthylamino-5-carboxamidofurane
2-(2-hydroxéthyl)amino-5-carboxamidofurane
2-(pyrrolidin-1-yl)-5-carboxamidofurane
2-amino-5-[N,N-diméthyl-carboxamido]-furane
2-méthylamino-5-[N, N-diméthyl-carboxamido]-furane
2-(2-hydroxéthyl)amino-5-[N,N-diméthyl-carboxamido]-furane
2-(pyrrolidin-1-yl)-5-[N,N-diméthyl-carboxamido]-furane
2-amino-5-[N-phényl-carboxamido]-furane
2-méthylamino-5-[N-phényl-carboxamido]-furane
2-(2-hydroxéthyl)amino-5-[N-phényl-carboxamido]-furane
2-(pyrrolidin-1-yl)-5-[N-phényl-carboxamido]-furane
2-amino-5-[N-(pyrazol-5-yl)-carboxamido]-furane
2-méthylamino-5-[N-(pyrazol-5-yl)-carboxamido]-furane
2-(2-hydroxéthyl)amino-5-[N-(pyrazol-5-yl)-carboxamido]-furane
2-(pyrrolidin-1-yl)-5-[N-(pyrazol-5-yl)-carboxamido]-furane
2-amino-5-[N-(1-méthylpyrazol-5-yl)-carboxamido]-furane
2-méthylamino-5-[N-(1-méthyl pyrazol-5-yl)-carboxamido]-furane
2-(2-hydroxéthyl)amino-5-[N-(1-méthyl pyrazol-5-yl)-carboxamido]-furane
2-(pyrrolidin-1-yl)- 5-[N-(1-méthyl pyrazol-5-yl)-carboxamido]-furane
2-amino-5-[N-(thiazol-2-yl)-carboxamido]-furane
2-méthylamino-5-[N-(thiazol-2-yl)-carboxamido]-furane
2-(2-hydroxéthyl)amino-5-[N-(thiazol-2-yl)-carboxamido]-(furane
2-(pyrrolidin-1-yl)- 5-[N-(thiazol-2-yl)-carboxamido]-furane
2-amino-5-[N-(triazol-3-yl)-carboxamido]-furane
2-méthylamino-5-[N-(triazol-3-yl)-carboxamido]-furane
2-(2-hydroxéthyl)amino-5-[N-(triazol-3-yl)-carboxamido]-furane
2-(pyrrolidin-1-yl )-5-[N-(triazol-3-yl)-carboxamido]-furane
2-amino-5-[N-(pyrid-2-yl)-carboxamido]-furane
2-méthylamino-5-[N-(pyrid-2-yl)-carboxamido]-furane
2-(2-hydroxéthyl)amino-5-[N-(pyrid-2-yl)-carboxamido]-furane
2-(pyrrolidin-1-yl)-5-[N-(pyrid-2-yl)-carboxamido]-furane
2-amino-5-[N-(piperidin-1-yl)-carboxamido]-furane
2-méthylamino-5-[N-(piperidin-1-yl)-carboxamido]-furane
2-(2-hydroxéthyl)amino-5-[N-(piperidin-1-yl)-carboxamido]-furane
2-(pyrro)idin-1-yl)-5-[N-(piperidin-1-yl)-carboxamido]-furane
2-amino-3-chloro-5-carboxamidofurane
2-méthylamino-3-chloro-5-carboxamidofurane
2-(2-hydroxéthyl)amino-3-chloro-5-carboxamidofurane
2-(pyrrolidin-1-yl)-3-chloro-5-carboxamidofurane
2-amino-3-chloro-5-[N,N-diméthyl-carboxamido]-furane
2-méthylamino-3-chloro-5-[N,N-diméthyl-carboxamido]-furane
2-(2-hydroxéthyl)amino-3-chloro-5-[N,N-diméthyl-carboxamido]-furane
2-(pyrrolidin-1-yl)-3-chloro-5-[N,N-diméthyl-carboxamido]-furane
2-amino-3-chloro-5-[N-phényl-carboxamido]-furane
2-méthylamino-3-chloro-5-[N-phényl-carboxamido]-furane
2-(2-hydroxéthyl)amino-3-chloro-5-[N-phényl-carboxamido]-furane
2-(pyrrolidin-1-yl)-3-chloro-5-[N-phényl-carboxamido]-furane
2-amino-3-chloro-5-[N-(pyrazol-5-yl)-carboxamido]-furane
2-méthylamino-3-chloro-5-[N-(pyrazol-5-yl)-carboxamido]-furane
2-(2-hydroxéthyl)amino-3-chloro-5-[N-(pyrazol-5-yl)-carboxamido]-furane
2-(pyrrolidin-1-yl)-3-chloro-5-[N-(pyrazol-5-yl)-carboxamido]-furane
2-amino-3-chloro-5-[N-(thiazol-2-yl)-carboxamido]-furane
2-méthylamino-3-chloro-5-[N-(thiazol-2-yl)-carboxamido]-furane
2-(2-hydroxéthyl)amino-3-chloro-5-[N-(thiazol-2-yl)-carboxamido]-furane
2-(pyrrolidin-1-yl)-3-chloro-5-[N-(thiazol-2-yl)-carboxamido]-furane
ainsi que leurs sels d'addition avec un acide ou une base.

16. Composition selon l'une quelconque des revendications 1 à 15 dans laquelle le coupleur de formule (I) est choisi parmi le 2-amino-5-[N-(thiazol-2-yl)-carboxamido]-furane et ses sels d'addition

17. Composition selon l'une quelconque des revendications 1 à 16 dans laquelle la ou les bases d'oxydation sont choisies parmi les paraphénylènediamines, les bis-phénylalkylènediamines, les para-aminophénols, les ortho-aminophénols, les bases hétérocycliques et leurs sels d'addition.

18. Composition selon l'une quelconque des revendications 1 à 17 dans laquelle la ou les bases d'oxydation sont chacune présentes en quantité comprise 0,001 et 10 % en poids du poids total de la composition tinctoriale.

19. Composition selon l'une quelconque des revendications 1 à 18 comprenant un ou plusieurs coupleurs additionnels choisis parmi les métaphénylènediamines, les méta-aminophénols, les métadiphénols, les coupleurs naphtaléniques et les coupleurs hétérocycliques autres que ceux de formule (I) et leurs sels d'addition.

20. Composition selon l'une quelconque des revendications 1 à 19 dans laquelle le ou les coupleurs sont présents chacun en quantité comprise entre 0,001 et 10 % en poids du poids total de la composition tinctoriale.

21. Procédé de teinture d'oxydation des fibres kératiniques **caractérisé en ce qu'**on applique sur les fibres une composition telle que définie à l'une quelconque des revendications 1 à 20 en présence d'un agent oxydant pendant un temps suffisant pour révéler la couleur souhaitée.

22. Procédé selon la revendication 21 dans lequel l'agent oxydant est choisi parmi le peroxyde d'hydrogène, le peroxyde d'urée, les bromates de métaux alcalins, les persels, les peracides et les enzymes oxydases.

23. Procédé selon la revendication 21 ou 22 dans lequel l'agent oxydant est mélangé au moment de l'emploi.

24. Procédé selon la revendication 21 dans lequel l'agent oxydant est appliqué sur les fibres simultanément ou séquentiellement à la composition telle que définie selon l'une quelconque des revendications 1 à 20 sous forme d'une composition oxydante.

25. Dispositif à plusieurs compartiments ou "kit" de teinture à plusieurs compartiments, dans lequel un premier compartiment tinctoriale contient une composition telle que définie à l'une quelconque des revendications 1 à 20 et un deuxième compartiment contient un agent oxydant.

26. Utilisation d'au moins un composé du type furane tel que défini selon l'une quelconque des revendications 1 à 16 pour la teinture par oxydation des fibres kératiniques.

27. Composés du type furane de formule générale (I)
dans laquelle R1, R2, R3 et X sont tels que définis aux revendications 1 à 16 et R4 représente
- un radical phényle éventuellement substitué
- un radical hétéroaromatique éventuellement substitué choisi parmi un furyle, thiényle, oxazole, imidazole, thiazole, isoxazole, triazole, thiadiazole, pyridyle, pyrimidyle, pyrazinyle, pyridazinyle ;
- un radical NR₁₅R₁₆ ;
- un radical alkyle en C₂-C₈ linéaire ou ramifié pouvant être substitué par un ou plusieurs radicaux hydroxy, alcoxy OR₁₀, carboxy, carboxamido, alkylsulfonyle, , alklsulfoxyde, alkylsulfonamido, NR₂₅R₂₆,
- R₁₅, R₁₆, R₂₅ et R₂₆ représentent indépendamment l'un de l'autre un atome d'hydrogène, un radical phényle éventuellement substitué, un radical hétéroaromatique éventuellement substitué, un radical alkyle en C₁-C₆ éventuellement substitué par un ou plusieurs radicaux choisis dans le groupe constitué par un hydroxy, un alcoxy en C₁-C₂, un alkyl(C₁-C₄)sulfonamido, un carboxy, un carboxamido, un alkyl(C₁-C₄)sulfonyle, un alkyl(C₁-C₄)sulfoxyde, un amino, un (di)alkylamino, un (poly)hydroxyalkylamino en C₂-C₄,
- R₁₀ représente un radical alkyle en C₁-C₄ éventuellement substitué par un ou plusieurs radicaux choisis parmi les radicaux hydroxy, alcoxy en C₁-C₂, amino, (di)(C₁-C₄)alkylamino, (poly)hydroxyalkylamino en C₂-C₄.

28. Dérivés intermediaires nitro de formules : 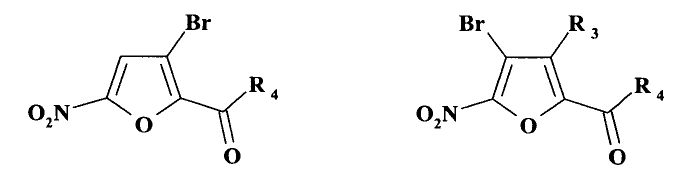 dans laquelle les radicaux R3 et R4 sont tels que définis selon l'une quelconque des revendications 1 à 14.
